# EUROPÄISCHE PATENTSCHRIFT

(11) **EP 2 601 896 B1**
(45) Veröffentlichungstag und Bekanntmachung des Hinweises auf die Patenterteilung: **07.10.2015**
(21) Anmeldenummer: 12194039.9
(22) Anmeldetag: 23.11.2012
(51) Int. Cl.: A61B 17/3203

(54) **Flüssigkeitsstrahlskalpell**
Fluid jet scalpel
Scalpel à jet liquide

(30) Priorität: 05.12.2011 DE 102011087748; 10.10.2012 US 201261711881 P
(43) Veröffentlichungstag der Anmeldung: 12.06.2013
(73) Patentinhaber: Deutsches Zentrum für Luft- und Raumfahrt e.V., 51147 Köln (DE)
(72) Erfinder: Bahls, Thomas, 86947 Weil (DE); Fröhlich, Florian Alexander, 82110 Germering (DE)
(74) Vertreter: Von Kreisler Selting Werner - Partnerschaft von Patentanwälten und Rechtsanwälten mbB

(56) Entgegenhaltungen:
- WO-A1-02/34153
- WO-A1-2011/141775
- DE-A1- 10 240 654
- JP-A- 2010 053 767
- US-A1- 2009 227 998
- US-A1- 2010 082 053
- ZHENG Y P ET AL: "Ultrasound elastomicroscopy using water jet and osmosis loading: Potentials for assessment for articular cartilage", ULTRASONICS, IPC SCIENCE AND TECHNOLOGY PRESS LTD. GUILDFORD, GB, vol. 44, 22 December 2006 (2006-12-22), pages e203-e209, XP025009170, ISSN: 0041-624X, DOI: 10.1016/J.ULTRAS.2006.06.008 [retrieved on 2006-12-22]

## Beschreibung

Die Erfindung betrifft ein Flüssigkeitsstrahlskalpell.

Flüssigkeitsstrahlskalpelle werden im medizinischen Bereich, beispielsweise in der Wasserstrahlchirurgie, verwendet.

Die zunehmend von Chirurgen verwendete Wasserstrahlchirurgie bietet die Möglichkeit, Weichgewebe selektiv zu trennen. So kann beispielsweise Lebergewebe getrennt werden, wobei Blutgefäße und Nerven unbeschädigt bleiben. Um das Gewebe zu trennen wird eine Düse, aus der Wasser mit hohem Druck austritt, in geringem Abstand zur Gewebeoberfläche bewegt. Das weiche Gewebe wird ausgespült und abgesaugt, die Blutgefäße und Nerven bleiben unverletzt, bilden aber nun ungeschützte Brücken zwischen den zu trennenden Organteilen. Sollen die Nerven und Blutgefäße auch durchtrennt werden, ist dazu ein Arbeitsschritt notwendig, in dem zum Beispiel mit einem zusätzlichen Werkzeug (z. B. Elektroskalpell oder Schere) gezielt die verbliebenen Stränge gekappt werden. Bei den Blutgefäßen wird zusätzlich vor dem Schneiden der Blutfluss unterbrochen (z. B. Elektroskalpell oder Klammern). Der gesamte Eingriff erfolgt bislang ausschließlich unter Sichtkontrolle.

Beim Verwenden eines Flüssigkeitsstrahlskalpells können durch die Düse oder den Instrumentenschaft ungewollt Gefäße beschädigt werden. Handelt es sich hierbei um Blutgefäße, kann die entstehende Blutung die Übersicht im Operationsgebiet drastisch verschlechtern. Außerdem kann dies frühzeitig zur verminderten Durchblutung eines abzutrennenden Organteils führen.

US 2010/082053 A1 beschreibt ein Flüssigkeitsstrahlskalpell mit einer Vorrichtung zum Messen des Abstandes zwischen der Düse und dem zu manipulierenden Gewebe.

Eine ähnliches Flüssigkeitsstrahlskalpell ist in DE 102 40 654 A1 beschrieben.

WO 023 4153, auf welchem der Oberbegriff von Anspruch 1 beruht, beschreibt ein Flüssigkeitsstrahlskalpell mit dynamischer Bildgebung zur Erfassung von nicht-knöchernen Strukturer, welche auf Ultraschall basiert.

Aufgabe der Erfindung ist es, ein Flüssigkeitsstrahlskalpell bereitzustellen, durch das eine Verletzung von Gewebe vermieden werden kann.

Die Lösung der Aufgabe erfolgt erfindungsgemäß durch die Merkmale des Vorrichtungsanspruchs 1.

Das erfindungsgemäße Flüssigkeitsstrahlskalpell weist eine Düse zum Ausgeben der unter Druck stehenden Flüssigkeit in Richtung des zu manipulierenden Gewebes auf. Bei dieser Flüssigkeit kann es sich beispielsweise um Wasser handelt. Ferner weist das erfindungsgemäße Flüssigkeitsstrahlskalpell eine Abstandsmessvorrichtung zum Messen des Abstandes zwischen der Düse und dem zu manipulierenden Gewebe auf. Bei dem zu manipulierenden Gewebe kann es sich um Gewebe handeln, das durch das Flüssigkeitsstrahlskalpell geschnitten werden soll.

Andererseits kann es sich um zu schonendes Gewebe handeln, das heißt, solches Gewebe, das durch das Flüssigkeitsstrahlskalpell nicht geschnitten werden kann oder soll. Hierbei kann es sich beispielsweise um Nervenbahnen, Blutgefäße, Knorpel oder ähnliche Strukturen handeln.

Durch das erfindungsgemäße Flüssigkeitsstrahlskalpell ist es somit möglich, eine Information darüber zu erhalten, wie weit die Düse von dem zu manipulierenden Gewebe entfernt ist. Mit dieser gemessenen Entfernung kann insbesondere ein Durchstoßen von Gewebe, welches aufgrund der selektiven Wirkung des Flüssigkeitsstrahls nicht abgetragen wird (z. B. Blutgefäße und Nerven), vermieden werden. In diesem Fall kann eine Warnmeldung ausgegeben werden. Ferner ist es möglich, eine bestimmte Bewegung des Flüssigkeitsstrahlskalpells zu unterbinden. Die Sicherheit beim Betrieb des Flüssigkeitsstrahlskalpells kann somit erfindungsgemäß erhöht werden.

Es ist erfindungsgemäß vorgesehen, dass durch die Abstandsmessvorrichtung ein Einkoppeln einer Abstandsmesswelle in den Flüssigkeitsstrahl erfolgt. Bei der Abstandsmesswelle handelt es sich um eine Ultraschallwelle, durch die nach dem Prinzip eines Ultraschallgeräts der Abstand zwischen dem Gewebe und der Düse gemessen wird. Die Abstandsmessvorrichtung ist somit eine akustische Abstandsmessvorrichtung.

Unter einem Einkoppeln des Messstrahls in den Flüssigkeitsstrahl wird verstanden, dass der Flüssigkeitsstrahl als Übertragungsmedium verwendet wird. Der Ultraschall benötigt ein Übertragungsmedium, um sich in den Körper des Patienten fortpflanzen zu können. Dieses Übertragungsmedium kann erfindungsgemäß der Flüssigkeitsstrahl sein.

Durch die genannten Merkmale kann die Abstandsmessung zwischen der Düse und exakt derjenigen Stelle des Gewebes erfolgen, die vom Flüssigkeitsstrahl manipuliert wird. Die Abstandsmessung erfolgt bevorzugt berührungslos. Dies bedeutet, dass, abgesehen von der aus der Düse austretenden Flüssigkeit, keine mechanische Verbindung zwischen der Düse/der Abstandsmessvorrichtung und dem Gewebe besteht.

Es ist weiterhin bevorzugt, dass das Flüssigkeitsstrahlskalpell eine Vorrichtung zum Erfassen der räumlichen Position und Lage der Düse aufweist. Hierbei kann es sich beispielsweise um ein Trackingsystem handeln. Ferner kann das Flüssigkeitsstrahlskalpell eine Berechnungseinrichtung zum Berechnen eines Modells des zu manipulierenden Gewebes aufweisen oder mit einer solchen datentechnisch verbunden sein. Die Berechnung des Modells erfolgt basierend auf dem gemessenen Abstand zwischen der Düse und dem Gewebe und der erfassten Position und Lage der Düse. Ferner kann das Flüssigkeitsstrahlskalpell eine Warnvorrichtung, beispielsweise eine akustische und/oder optische Warnvorrichtung zum Ausgeben eines Warnsignals bei Unterschreitung eines vorbestimmten Abstandes zwischen der Düse und dem zu schonenden Gewebe aufweisen.

Das erfindungsgemäße Flüssigkeitsstrahlskalpell kann sämtliche Merkmale aufweisen, die in Zusammenhang mi dem im Folgenden beschriebenen Verfahren zum Betreiben des Flüssigkeitsstrahlskalpells beschrieben werden.

Ein beispielhaftes Verfahren zum Betreiben eines Flüssigkeitsstrahlskalpells umfasst den Schritt:
Messen des Abstandes zwischen der Düse des Flüssigkeitsstrahlskalpells und dem zu manipulierenden Gewebe

Bevorzugt erfolgt zusätzlich ein Erfassen der räumlichen Position und Lage der Düse sowie ein Berechnen eines Modells des zu manipulierenden Gewebes, basierend auf dem gemessenen Abstand zwischen der Düse und dem Gewebe und der erfassten Position und Lage der Düse.

Weiterhin kann als zusätzlicher Schritt eine Warnmeldung ausgegeben werden, wenn der Abstand zwischen der Düse und dem zu schonenden Gewebe einen vorbestimmten Schwellwert unterschreitet. Insbesondere kann angezeigt werden, welche Stelle des Flüssigkeitsstrahlskalpells zu nah an das Gewebe herangekommen ist. Dies kann beispielsweise auf einer Anzeigevorrichtung erfolgen, durch die ein Chirurg einen Telemanipulationsroboter steuert. Beispielsweise kann angezeigt werden, dass das Wasserstrahlskalpell mit der Düse, seinem Schaft oder einem anderen Element zu nah an einen kritischen Bereich herangekommen ist.

Es ist weiterhin bevorzugt, dass der Abstand zwischen der Düse und dem Gewebe kontinuierlich gemessen wird. Unter "kontinuierlich" wird auch ein Messen in kurzen zeitlichen Abständen, beispielsweise Bruchteilen einer Sekunde, verstanden. Somit kann sichergestellt werden, dass die Düse zu keinem Zeitpunkt während einer Operation zu nah an das zu manipulierende Gewebe herankommt.

In einer bevorzugten Ausführungsform wird ein Modell des zu schonenden Gewebes durch das beispielhafte Verfahren erstellt. Aufgrund der Selektivität beim Schneiden mit einem Flüssigkeitsstrahlskalpell werden von diesem bestimmte Arten von Gewebe (Blutgefäße, Nervenbahnen usw.) nicht geschnitten. So kann es vorkommen, dass nach einem mehrmaligen Bewegen der Düse über ein zu schneidendes Gewebe (beispielsweise über Lebergewebe) das zu schneidende Gewebe durch den Flüssigkeitsstrahl geschnitten und weggespült wurde, während beispielsweise eine sich an dieser Stelle befindliche Blutbahn verschont geblieben ist. Durch das wiederholte Abfahren dieser Stelle mit dem Flüssigkeitsstrahlskalpell wurde somit diese Blutbahn oder allgemeiner, das zu schonende Gewebe, freigelegt, da sich um dieses zu schonende Gewebe herum kein anderes Gewebe mehr befindet. Wird nun beim erneuten Abfahren derselben Trajektorie der Abstand zwischen der Düse und dem Gewebe gemessen, so wird im Bereich des zu schonenden Gewebes ein sprunghafter Abfall des gemessenen Abstandes verzeichnet und anschließend nach dem zu schonenden Gewebe ein sprunghafter Anstieg dieses Abstandes. Anders ausgedrückt, weist das geschnittene Gewebe einen größeren Abstand zu der Düse auf, als das freigelegte zu schonende Gewebe, das sich in sprunghafter Weise näher an der Düse befindet. Diese sprunghafte Annäherung kann durch den gemessenen Abstand zwischen der Düse und dem Gewebe detektiert werden. In Abhängigkeit der gerade durchgeführten Operation wird ein Schwellwert für diesen sprunghaften Abfall und Anstieg des Abstandes festgelegt. Sofern der sprunghafte Abfall und Anstieg des Abstandes diesen festgelegten Schwellwert überschreiten, wird für die Berechnung des Modells des Gewebes angenommen, dass sich an dieser Stelle zu schonendes Gewebe, beispielsweise ein Blutgefäß, befindet, das aufgrund der Selektivität beim Schneiden mit dem Flüssigkeitsstrahlskalpell von diesem nicht geschnitten wurde und somit eine freigelegte, zu schützende Gewebebrücke bildet. Diese wird sodann in das erstelle Gewebemodell aufgenommen und kann im weiteren Verlauf der Operation entsprechend berücksichtigt werden. Insbesondere ist es möglich, an dieser Stelle eine softwareseitige Beschränkung des Bewegungsbereiches der Düse vorzunehmen, sofern diese beispielsweise durch einen medizinischen Roboter gesteuert wird, so dass die freigelegte Gewebebrücke geschützt werden kann.

Durch die genannten Verfahrensschritte ist es weiterhin möglich, die Breite des detektierten zu schonenden Gewebes zu berücksichtigen, so dass beispielsweise nur dann davon ausgegangen wird, dass es sich um zu schonendes Gewebe handelt, wenn sich der sprunghafte Abfall des Abstandes zwischen der Düse und dem Gewebe über eine definierte Mindestbreite erstreckt. Anderenfalls kann zum Beispiel von einem Messfehler ausgegangen werden.

Wird auf diese Weise zum Beispiel ein zu schonendes Blutgefäß detektiert, ist es möglich, den Schneidevorgang mit dem Flüssigkeitsstrahlskalpell zu unterbrechen und dieses Blutgefäß durch Verfahren, die aus dem Stand der Technik bekannt sind, zu trennen. Hierfür wird vor dem Trennen der Blutfluss, beispielsweise durch ein Elektroskalpell oder Klammern, unterbrochen. Anschließend kann das zu schonende Gefäß durchtrennt werden. Diese Trennung des zu schonenden Gefäßes findet anschließend Berücksichtigung in dem erstellten Gewebemodell, indem beispielsweise dieses zu schonende Gewebe als solches wieder aus dem Gewebemodell entfernt wird.

In einer bevorzugten Ausführungsform des erfindungsgemäßen Verfahrens wird das Flüssigkeitsstrahlskalpell durch einen Roboter geführt. Die räumliche Position und Lage der Düse kann in dieser Ausführungsform, basierend auf den Messwerten von Sensoren des Roboters, berechnet werden. Es werden somit für die Bestimmung der räumlichen Position und Lage der Düse Messwerte verwendet, die durch die Sensoren des Roboters, die sich beispielsweise in oder an seinen Gelenken befinden, ohnehin vorhanden sind. Das Vorsehen zusätzlicher Sensoren oder beispielsweise eines separaten Trackingsystems ist somit nicht mehr notwendig.

Weiterhin kann bei einer Führung des Flüssigkeitsstrahlskalpells durch einen Roboter eine zu starke Annäherung der Düse an das zu manipulierende Gewebe durch eine insbesondere softwareseitige Beschränkung des Bewegungsraums des Roboters unterbunden werden. Unter einer zu starken Annäherung wird eine Annäherung verstanden, bei der der Abstand zwischen der Düse und dem zu manipulierenden Gewebe einen vorbestimmten Schwellwert unterschreitet. Eine softwareseitige Beschränkung des Bewegungsraums des Roboters ist eine Beschränkung, die nicht durch hardwareseitige Beschränkungen, beispielsweise Beschränkungen des Verschwenkbereichs von Gelenken, verursacht wird. Vielmehr handelt es sich hierbei um eine künstlich erzeugte Beschränkung, die durch die Steuervorrichtung des Roboters vorgenommen wird und jederzeit verändert bzw. wieder entfernt werden kann.

Die zuletzt beschriebenen Verfahrensschritte bilden auch eine gute Grundlage für mögliche teilautonome oder autonome Prozessschritte, die durch einen medizinischen Roboter durchgeführt werden sollen.

Neben einer Anwendung im Bereich Medizintechnik kann das erfindungsgemäße Flüssigkeitsstrahlskalpell auch in anderen Bereichen, beispielsweise in der Industrie, Anwendung finden, in denen ein bestimmter Mindestabstand zu den zu manipulierenden Objekten eingehalten werden muss.

Im Folgenden wird eine bevorzugte Ausführungsform der Erfindung anhand einer Figur erläutert.

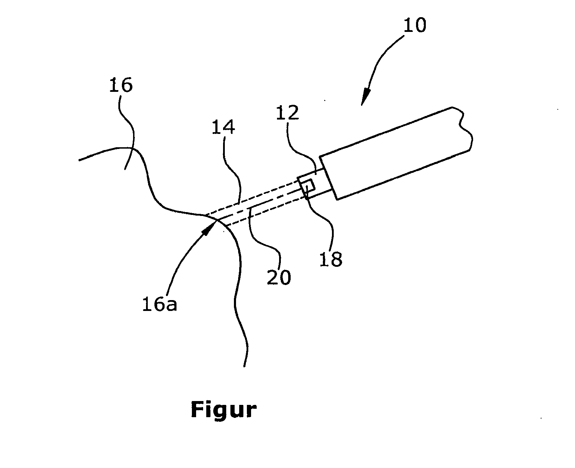

Die Figur zeigt eine schematische Ansicht einer Ausführungsform des erfindungsgemäßen Flüssigkeitsstrahlskalpells.

Ein Wasserstrahlskalpell 10 weist eine Düse 12 auf, die an seinem distalen Ende angeordnet ist. Ebenfalls an seinem distalen Ende angeordnet ist eine Abstandsmessvorrichtung 18, die beispielswiese koaxial zur Düse 12 angeordnet sein kann. Aus der Düse 12 wird ein Wasserstrahl 14 in Richtung des zu manipulierenden Gewebes 16 ausgegeben. Der Wasserstrahl 14 trifft an der Stelle 16a auf das Gewebe 16. Die Abstandsmessvorrichtung 18 strahlt einen Abstandsmessstrahl 20 aus, durch den der Abstand zwischen der Düse 12 und dem Punkt 16a gemessen wird.

## Patentansprüche

1. Flüssigkeitsstrahlskalpell, mit
einer Düse (12) zum Ausgeben der unter Druck stehenden Flüssigkeit (14) in Richtung eines zu manipulierenden Gewebes (16) und
einer Abstandsmessvorrichtung (18) zum Messen des Abstandes zwischen der Düse (12) und dem zu manipulierenden Gewebe (16),
**dadurch gekennzeichnet, dass**
durch die Abstandsmessvorrichtung (18) ein Einkoppeln einer Abstandsmesswelle (20) in den Flüssigkeitsstrahl (14) erfolgt, so dass die Abstandsmessung zwischen der Düse (12) und exakt der Stelle (16a) des Gewebes (16) erfolgt, die vom Flüssigkeitsstrahl (14) manipuliert wird, wobei die Abstandsmessung berührungslos erfolgt, indem abgesehen von der aus der Düse (12) austretenden Flüssigkeit keine mechanische Verbindung zwischen der Düse (12) / der Abstandsmessvorrichtung (18) und dem Gewebe (16) besteht,
wobei die Abstandsmessvorrichtung (18) eine Ultraschallmessvorrichtung ist und die Abstandsmesswelle (20) eine akustische Abstandsmesswelle ist.

2. Flüssigkeitsstrahlskalpell nach Anspruch 1, **gekennzeichnet durch** die folgenden Komponenten:
- eine Vorrichtung zum Erfassen der räumlichen Position und Lage der Düse (12), insbesondere eines Tracking-Systems,
- einer Berechnungseinrichtung zum Berechnen eines Modells des zu manipulierenden Gewebes (16), basierend auf dem gemessenen Abstand zwischen der Düse (12) und dem Gewebe (16) und der erfassten Position und Lage der Düse (12),
- einer Warnvorrichtung zum Ausgeben eines Warnsignals bei Unterschreitung eines vorbestimmten Abstandes zwischen der Düse (12) und dem Gewebe (16).

## Claims

1. A liquid jet scalpel comprising
a nozzle (12) for discharge of pressurized liquid (14) in the direction of tissue (16) that is to be manipulated, and
a distance measuring device (18) for measuring the distance between the nozzle (12) and the tissue (16) that is to be manipulated,
**characterized in that**
the distance measuring device (18) is operative to couple a distance measuring wave (20) into the liquid jet (14) in such a manner that the distance measurement is performed between the nozzle (12) and exactly that site (16a) of the tissue (16) which is manipulated by the liquid jet (14), the distance measurement being performed in a contactless manner **in that**, except for the liquid issuing from the nozzle (12), no mechanical connection exists between the nozzle (12) / the distance measuring device (18) and the tissue (16),
wherein the distance measuring device (18) is an ultrasonic distance measuring device and the distance measuring wave (20) is an acoustic distance measuring wave.

2. The liquid jet scalpel according to claim 1, **characterized by** the following components:
- a device for detecting the spatial position and orientation of the nozzle (12), said device preferably being a tracking system,
- a computing device for computing a model of the tissue (16) that is to be manipulated, said computation being performed on the basis of the measured distance between the nozzle (12) and the tissue (16) and of the detected position and orientation of the nozzle (12),
- a warning device for emitting a warning signal in case that said measured distance falls short of a predetermined distance between the nozzle (12) and the tissue (16).

## Revendications

1. Bistouri à jet de liquide, comprenant
une buse (12) destinée à éjecter le liquide sous pression (14) en direction d'un tissu à manipuler (16) et
un dispositif de mesure de la distance (18) destiné à mesurer la distance entre la buse (12) et le tissu à manipuler (16),
**caractérisé en ce que**,
sous l'action du dispositif de mesure de la distance (18), il se produit une injection d'une onde de mesure de la distance (20) dans le jet de liquide (14), de sorte que la mesure de la distance s'effectue entre la buse (12) et exactement l'endroit (16a) du tissu (16) qui est manipulé par le jet de liquide (14), la mesure de la distance s'effectuant sans contact, puisqu'à l'exception du liquide qui sort de la buse (12), il n'y a aucune liaison mécanique entre la buse (12), le dispositif de mesure de la distance (18) et le tissu (16),
le dispositif de mesure de la distance (18) étant un dispositif de mesure à ultrasons et **en ce que** l'onde de mesure de la distance (20) est une onde de mesure de distance acoustique.

2. Bistouri à jet de liquide selon la revendication 1, **caractérisé par** les composants suivants :
- un dispositif pour détecter la position spatiale et l'orientation de la buse (12), qui fait en particulier partie d'un système de poursuite,
- un dispositif de calcul servant à calculer un modèle du tissu (16) à manipuler, en se basant sur la distance mesurée entre la buse (12) et le tissu (16) et sur la position et l'orientation détectées de la buse (12),
- un dispositif d'alarme servant à émettre un signal d'alarme en cas de passage sous une distance prédéterminée entre la buse (12) et le tissu (16).
